(19) **Europäisches Patentamt / European Patent Office / Office européen des brevets**

(11) **EP 4 319 642 B1**

(12) **FASCICULE DE BREVET EUROPEEN**

(45) Date de publication et mention de la délivrance du brevet:
**03.06.2026 Bulletin 2026/23**

(21) Numéro de dépôt: **22720947.5**

(22) Date de dépôt: **04.04.2022**

(51) Classification Internationale des Brevets (IPC):
**A61B 7/00** *(2006.01)*

(52) Classification Coopérative des Brevets (CPC):
**A61B 7/003**

(86) Numéro de dépôt international:
**PCT/EP2022/058849**

(87) Numéro de publication internationale:
**WO 2022/214422 (13.10.2022 Gazette 2022/41)**

(54) **PROCÉDÉ DE DÉTERMINATION DE PHASES RESPIRATOIRES DANS UN SIGNAL ACOUSTIQUE, PRODUIT PROGRAMME D'ORDINATEUR, MÉDIUM DE STOCKAGE ET DISPOSITIF CORRESPONDANT**

**VERFAHREN ZUR BESTIMMUNG VON ATEMPHASEN IN EINEM AKUSTISCHEN SIGNAL, COMPUTERPROGRAMMPRODUKT, SPEICHERMEDIUM UND ENTSPRECHENDE VORRICHTUNG**

**METHOD FOR DETERMINING RESPIRATORY PHASES IN AN ACOUSTIC SIGNAL, COMPUTER PROGRAM PRODUCT, STORAGE MEDIUM AND CORRESPONDING DEVICE**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **06.04.2021 FR 2103511**

(43) Date de publication de la demande:
**14.02.2024 Bulletin 2024/07**

(73) Titulaires:
• **Controle Instrumentation et Diagnostic Electroniques - CIDELEC**
**49130 Sainte-Gemmes-sur-Loire (FR)**
• **Ecole Superieure Electronique de l'Ouest**
**49100 Angers (FR)**

(72) Inventeurs:
• **FREYCENON, Nathalie**
**79320 BRISSAC-LOIRE-AUBANCE (FR)**
• **LONGO, Roberto**
**49100 ANGERS (FR)**
• **MENIGOT, Sébastien**
**49107 ANGERS (FR)**

(74) Mandataire: **Vidon Brevets & Stratégie**
**16B, rue de Jouanet**
**BP 90333**
**35703 Rennes Cedex 7 (FR)**

(56) Documents cités:
EP-A1- 2 593 007  EP-B1- 2 593 007
US-A1- 2012 253 214  US-A1- 2012 253 216
US-A1- 2017 172 459

## Description

### Domaine technique

L'invention s'inscrit dans le domaine du traitement des signaux acoustiques, notamment des sons trachéaux ou pulmonaires captés sur un individu.

Plus précisément, l'invention concerne une technique de reconnaissance des phases respiratoires d'un individu à partir d'un signal acoustique représentatif de l'activité respiratoire de cet individu.

L'invention s'applique notamment, mais non exclusivement, dans le domaine de l'instrumentation acoustique dédiée à l'enregistrement et la surveillance de l'activité respiratoire d'individus, par exemple pour l'étude de pathologies respiratoires ou de troubles respiratoires durant le sommeil.

### Arrière-plan technologique

On s'attache plus particulièrement dans la suite de ce document à décrire la problématique existant dans le cadre de l'étude de l'activité respiratoire sur des patients atteints du « syndrome d'apnées du sommeil » (connu sous le nom de syndrome SAS). L'invention ne se limite bien sûr pas à ce contexte particulier d'application, mais présente un intérêt pour toute technique de reconnaissance de phases respiratoires devant faire face à une problématique proche ou similaire.

Le syndrome d'apnées du sommeil se manifeste par des interruptions répétées et incontrôlées de la respiration pendant le sommeil. Elles entrainent des micro-éveils non-conscients pouvant nuire à la qualité du sommeil. Il peut donc en résulter des somnolences diurnes, des difficultés de concentration ou de mémoire, voire des complications sur le plan cardio-vasculaire, sources éventuelles d'accidents corolaires tels que les accidents de la route ou du travail. Il importe donc de disposer d'un appareillage médical capable de détecter ce genre de pathologie, à des fins de dépistage et de traitement notamment.

Le diagnostic du syndrome d'apnée du sommeil repose principalement sur l'exploration de la ventilation de l'individu afin d'observer les mouvements de la respiration et l'évolution des voies aériennes supérieures au cours de son sommeil. Les apnées et hypopnées sont recherchées en analysant l'amplitude de signaux acoustiques obtenus par exemple lors d'une polygraphie ventilatoire ou d'une polysomnographie. Il est généralement recommandé d'utiliser un certain nombre d'instruments de mesure pour mesurer le comportement respiratoire de l'individu pendant son sommeil, tels que des lunettes nasales, des sangles thoraco-abdominales, un oxymètre de pouls, etc. Un logiciel d'analyse analyse les signaux enregistrés par les différents instruments de mesure afin d'identifier d'éventuels évènements pathologiques, tels que des apnées et des hypopnées. D'autres instruments, tels que des capteurs de son trachéal, peuvent être également utilisés de façon complémentaire pour étudier plus précisément certains paramètres associés aux phases d'inspiration et d'expiration de l'activité respiratoire de l'individu, et permettre d'affiner le diagnostic. En effet, les dernières études menées sur les cycles respiratoires obtenus à partir de signaux sonores trachéaux ont permis de mettre en avant un certain nombre d'indicateurs pathologiques dont la prise en considération s'avère pertinente dans la détection et l'évaluation des troubles respiratoires.

Toutefois, même s'il existe des techniques d'analyse de cycles respiratoires à partir de sons trachéaux, comme la technique non-invasive de détection de phases proposée par ZK Moussavi et Al. dans l'article scientifique « *Computerised acoustical respiratory phase detection without airflow measurement* » (Mars 2000), celles-ci manquent de précision. L'activité respiratoire d'un individu (composée typiquement de cycles respiratoires successifs, chaque cycle respiratoire comprenant une phase d'inspiration et une phase d'expiration) peut s'observer à partir des trois représentations suivantes : une représentation temporelle, une représentation fréquentielle et une représentation fréquence-intensité au cours du temps (représentation « tridimensionnelle »). Or il s'avère qu'il est souvent difficile de distinguer précisément la phase d'inspiration de la phase d'expiration d'un cycle respiratoire dans le domaine fréquentiel ou temporel d'un signal sonore trachéal, en particulier lorsque le signal capté se situe en dessous d'un certain seuil sonore (cas d'un individu au repos par exemple). Par ailleurs, de telles techniques connues ne fournissent qu'un nombre limité d'informations, relativement peu précises, sur l'activité respiratoire du **patient.** Le document EP2593007 divulgue un procédé de détermination de phases respiratoires dans un signal acoustique représentatif d'une activité respiratoire d'un individu selon la préambule de la revendication 1 et un dispositif selon la préambule de la revendication de la revendication 10.

Par ailleurs, certains profils respiratoires s'avèrent particulièrement complexes à traiter, par exemple en cas de ronflements ou encore en cas d'apnées au cours desquelles la diminution du signal acoustique s'accompagne de bruits liés aux efforts de l'individu pour faire entrer ou sortir de l'air via les voies respiratoires. D'autres bruits, tels que les déglutitions, la toux, les raclements de gorges, voire les changements de position de l'individu au cours de son sommeil compliquent la reconnaissance des phases respiratoires.

Il existe donc un vrai besoin de fournir une technique qui permette une reconnaissance efficace des phases respiratoires d'un individu dans un signal sonore trachéal, et plus généralement dans un signal acoustique représentatif d'une activité respiratoire. Il serait également particulièrement intéressant de fournir une telle technique qui soit peu invasive et facile à mettre en œuvre.

## Exposé de l'invention

Dans un mode de réalisation particulier de l'invention tel que défini dans la revendication indépendante 1, il est proposé un procédé de détermination de phases respiratoires dans un signal acoustique représentatif d'une activité respiratoire d'un individu, l'activité respiratoire étant définie par des cycles respiratoires chacun comprenant une phase d'inspiration et une phase d'expiration, le procédé est remarquable en ce qu'il comprend les étapes suivantes :

- détection d'intervalles de pause dans le signal acoustique ;
- détermination d'intervalles d'activité correspondant à des phases d'inspiration et d'intervalles d'activité correspondant à des phases d'expiration chacune par comparaison, pour chaque intervalle de temps, de la durée de l'intervalle de pause qui précède ledit intervalle de temps avec la durée de l'intervalle de pause qui suit ledit intervalle de temps.

Ainsi, le principe de l'invention repose sur une recherche dans le signal acoustique de pauses du signal pour en déduire les phases d'inspiration et d'expiration grâce à leur durée et leur position dans le signal. Cette nouvelle approche est astucieuse car, plutôt que de rechercher directement à traiter les phases d'inspiration et d'expiration comme dans l'état de l'art, la présente invention s'attache d'abord à reconnaître les pauses dans le signal, celles-ci étant plus aisées à reconnaître dans un signal acoustique. Ainsi, l'invention offre une technique de reconnaissance de phases respiratoires qui est plus efficace que les techniques de l'état de l'art. Par ailleurs, le fait de pouvoir déterminer les phases inspiratoires et expiratoires à l'aide uniquement d'un signal acoustique offre des perspectives d'examen allégé en matière d'équipement médical.

Selon un aspect particulier, le procédé met en œuvre un mécanisme de distinction parmi les intervalles de pause, entre un premier type d'intervalle de pause, dit pause courte, de durée inférieure à un deuxième type d'intervalle de pause, dit pause longue.

Selon une caractéristique particulière, le signal acoustique appartient au groupe comprenant : un signal acoustique trachéal ou un signal acoustique pulmonaire capté sur un individu.

Selon une mise en œuvre particulière du procédé, si à l'issue de l'étape de détection au moins une succession de deux intervalles de pause séparés dans le signal par un intervalle de temps, dit intervalle aberrant, de durée inférieure à un seuil de durée prédéterminé est identifiée, le procédé comprend une étape de remplacement, pour chaque succession détectée, de l'ensemble constitué par les deux intervalles de pause et ledit intervalle aberrant par un nouvel intervalle de pause. On optimise ainsi le mécanisme de reconnaissance des phases d'inspiration et d'expiration selon l'invention.

En effet, le seuil prédéterminé est dimensionné de sorte que lorsqu'un défaut ponctuel apparaît dans le signal entre deux intervalles de pause, celui-ci soit considéré comme faisant partie intégrante, avec les deux intervalles de pause, d'une même et unique pause. Cela permet de s'affranchir des bruits parasites contenus dans le signal qui viendrait perturber la reconnaissance des phases respiratoires.

Selon l'invention telle que définie dans la revendication indépendante 1, si à l'issue de l'étape de détermination au moins un intervalle de temps, dit intervalle indéterminé, répondant à un critère d'incohérence prédéterminé, est identifié, le procédé comprend les étapes suivantes, pour chaque intervalle indéterminé : -traitement de la portion de signal correspondant audit intervalle indéterminé, au moyen d'une méthode d'apprentissage automatique ; -association dudit au moins un intervalle indéterminé à une phase d'inspiration ou d'expiration en fonction des résultats de l'étape de traitement. On améliore ainsi davantage le mécanisme de reconnaissance des phases d'inspiration et d'expiration selon l'invention.

Selon un aspect particulier de l'invention, l'étape de détection d'intervalles de pause repose sur un traitement appartenant au groupe comprenant un traitement fréquentiel du signal acoustique, un traitement énergétique du signal acoustique. En effet, c'est en observant les espaces de représentation énergétique et temps-fréquence de signaux sonores qu'il a été constaté, de manière surprenante, que les pauses respiratoires sont plus aisément reconnaissables dans ces espaces de représentation que les phases d'inspiration et d'expiration elles-mêmes. Le mécanisme de reconnaissance de pauses selon l'invention peut être mis en œuvre soit par traitement fréquentiel du signal, soit par traitement énergétique du signal, ou soit par traitements simultanés fréquentiel et énergétique du signal, rendant ainsi le procédé encore plus fiable.

Selon une mise en œuvre particulière, le traitement fréquentiel comprend les étapes suivantes :

- application d'un filtre passe-haut anti-bruit audit signal pour retirer une première plage prédéfinie de fréquences audit signal et obtenir un signal filtré en fonction du temps ;
- segmentation dudit signal filtré pour obtenir une pluralité d'échantillons dudit signal filtré et, pour chaque échantillon, estimation de la densité spectrale moyenne de puissance dudit échantillon de manière à obtenir un signal fréquentiel représentant la fréquence centroïde en fonction du temps.
- application d'une fenêtre temporelle glissante de durée prédéterminée sur ledit signal fréquentiel et, pour chaque fenêtre :

    * détection d'un maximum fréquentiel du signal fréquentiel dans ladite fenêtre,
    * détermination d'un seuil de fréquence en fonction du maximum fréquentiel détecté dans ladite fenêtre ;

- mise en forme, à partir dudit signal fréquentiel, d'un premier signal logique transitoire de niveaux logiques définis en fonction des seuils de fréquence déterminés pour ledit signal fréquentiel, les intervalles de pauses étant détectés en fonction du premier signal logique transitoire.

Selon une mise en œuvre particulière, le traitement énergétique comprend les étapes :

- application d'un filtre passe-haut anti-bruit et d'un filtre passe-bas pour retirer audit signal respectivement une première et une deuxième plages prédéfinies de fréquences et obtenir un signal filtré en fonction du temps dans le domaine temporel ;
- application d'une première fenêtre temporelle glissante de durée prédéterminée sur ledit signal filtré et, pour chaque fenêtre temporelle, estimation de l'intensité acoustique dudit signal filtré par application d'une enveloppe à moyenne quadratique dans ladite fenêtre, de manière à transformer ledit signal filtré en un signal énergétique en fonction du temps ;
- application d'une deuxième fenêtre temporelle glissante de durée déterminée par autocorrélation sur ledit signal énergétique et, pour chaque fenêtre :

  * détection d'un minimum d'intensité du signal énergétique dans ladite fenêtre,
  * détermination d'un seuil d'intensité en fonction du minimum d'intensité détecté dans ladite fenêtre ;

- mise en forme, à partir dudit signal énergétique, d'un deuxième signal logique transitoire de niveaux logiques définis en fonction des seuils d'intensité déterminés pour ledit signal énergétique, les intervalles de pauses étant détectés en fonction du deuxième signal logique transitoire.

Selon un aspect particulièrement avantageux de l'invention, le procédé comprend une étape de mise en forme, par traitement desdits premier et deuxième signaux logiques par une fonction logique OU-Inclusif, d'un signal logique final dont les niveaux logiques hauts correspondent auxdits intervalles de pause détectés. Les traitements fréquentiel et énergétique pouvant être complémentaires, une prise en compte de ces deux traitements peut permettre de détecter des pauses qui n'auraient pas été détectées par la mise en œuvre d'un seul des deux traitements.

Selon une caractéristique particulière, le procédé comprend une étape consistant à, si à l'issue de l'étape de mise en forme, au moins une succession de deux intervalles de pause séparés par un intervalle aberrant détectée dans ledit signal logique final de durée inférieure au seuil de durée prédéterminée est identifiée : remplacer, pour chaque succession détectée dans ledit signal logique final, l'ensemble constitué par les deux intervalles de pause et ledit intervalle aberrant par un nouvel intervalle de pause. Cela facilite la distinction entre les pauses longues et les pauses courtes, et permet de fait une reconnaissance plus fiable des phases respiratoires.

Dans un autre mode de réalisation de l'invention tel que défini dans la revendication 8, il est proposé un produit programme d'ordinateur qui comprend des instructions de code de programme pour la mise en œuvre du procédé précité (dans l'un quelconque de ses différents modes de réalisation), lorsque ledit programme est exécuté sur un ordinateur.

Dans un autre mode de réalisation de l'invention tel que défini dans la revendication 9, il est proposé un médium de stockage lisible par ordinateur et non transitoire, stockant un programme d'ordinateur comprenant un jeu d'instructions exécutables par un ordinateur pour mettre en œuvre le procédé précité (dans l'un quelconque de ses différents modes de réalisation).

Dans un autre mode de réalisation de l'invention tel que défini dans la revendication 10, il est proposé un dispositif de détermination de phases respiratoires dans un signal acoustique représentatif d'une activité respiratoire d'un individu, l'activité respiratoire étant définie par des cycles respiratoires chacun comprenant une phase d'inspiration et une phase d'expiration, le dispositif étant caractérisé en ce qu'il comprend : - des moyens de détection d'intervalles de pause dans le signal acoustique ; - des moyens de détermination d'intervalles d'activité correspondant à des phases d'inspiration et d'intervalles d'activité correspondant à des phases d'expiration par comparaisons de la durée des intervalles de pause. Le dispositif de la revendication 10 comprend aussi des moyens de traitement de la portion de signal correspondant d'au moins un intervalle indéterminé au moyen d'une méthode d'apprentissage automatique, un intervalle indéterminé étant un intervalle de temps déterminé répondant à un critère d'incohérence prédéterminé et des moyens d'association dudit au moins un intervalle indéterminé à une phase d'inspiration ou d'expiration en fonction des résultats issus des moyens de traitement.

Avantageusement, le dispositif comprend des moyens de mise en œuvre des étapes qu'il effectue dans le procédé d'obtention tel que décrit précédemment, dans l'un quelconque de ses différents modes de réalisation.

## Liste des figures

D'autres caractéristiques et avantages de l'invention apparaîtront à la lecture de la description suivante, donnée à titre d'exemple indicatif et non limitatif, et des dessins annexés, dans lesquels :

- la figure 1 présente un organigramme d'un mode de réalisation particulier du procédé selon l'invention ;
- la figure 2 illustre de manière simplifiée le principe d'enregistrement d'une activité respiratoire d'un individu au moyen d'un capteur sonore trachéal ;
- la figure 3 présente un organigramme illustrant une implémentation particulière d'un traitement fréquentiel et d'un traitement énergétique du signal acoustique trachéal conforme à l'invention ;
- la figure 4 est une représentation graphique temporelle illustrant l'évolution de l'intensité d'un signal acoustique trachéal enregistré sur un individu ;
- la figure 5 est une représentation graphique temporelle illustrant le principe de traitement fréquentiel du signal acoustique selon l'invention ;
- la figure 6 est une représentation graphique temporelle de type chronogramme mettant en évidence les pauses respiratoires contenues dans signal acoustique par traitement fréquentiel ;
- la figure 7 est une représentation graphique temporelle illustrant le principe de traitement énergétique du signal acoustique selon l'invention ;
- la figure 8 est une représentation graphique temporelle de type chronogramme mettant en évidence les pauses respiratoires détectées dans signal acoustique par traitement énergétique ;
- la figure 9 représente un chronogramme final mettant en évidence les pauses respiratoires détectées dans signal acoustique à partir des traitements fréquentiel et énergétique ;
- la figure 10 représente un chronogramme final illustrant le principe de détermination des phases d'inspiration et d'expiration en fonction des pauses détectées dans le signal acoustique ;
- la figure 11 représente la structure simplifiée d'un dispositif mettant en œuvre le procédé selon un mode de réalisation particulier de l'invention.

**Description détaillée de l'invention**

Sur toutes les figures du présent document, les éléments et étapes identiques sont désignés par une même référence numérique.

Dans la suite de la description, on considère un exemple de mise en œuvre de l'invention dans le cadre d'un signal acoustique trachéal, c'est-à-dire d'un signal acoustique obtenu sur la trachée d'un individu pour réaliser un examen de l'activité respiratoire de celui-ci au cours du sommeil (par exemple pour diagnostiquer un syndrome d'apnées du sommeil). L'invention ne se limite bien sûr pas à ce contexte particulier, et peut s'appliquer à tout type de signaux acoustiques représentatifs d'une activité respiratoire d'un être vivant.

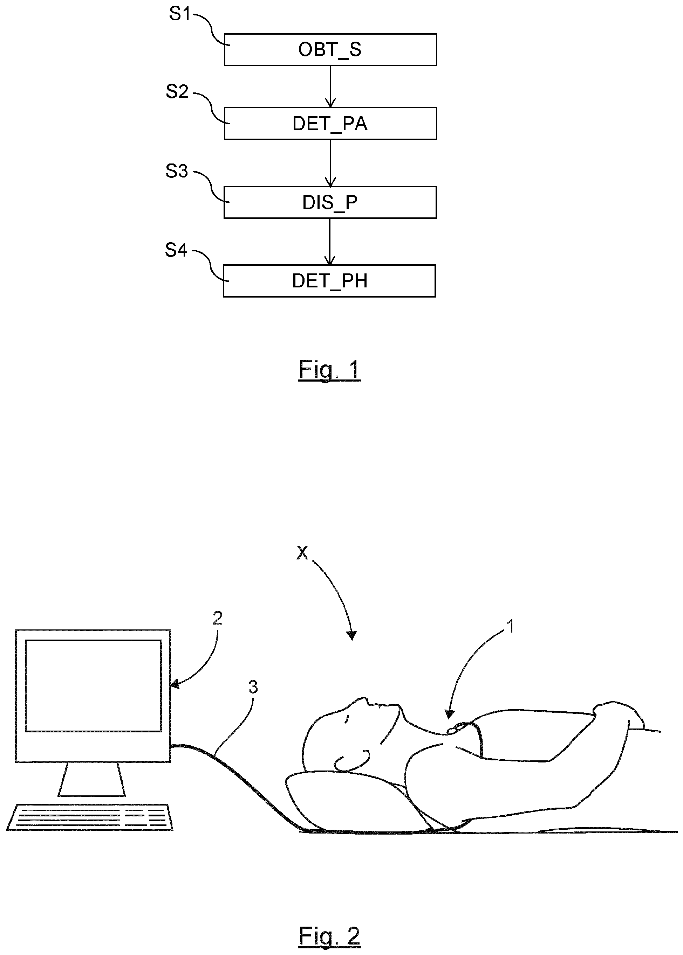

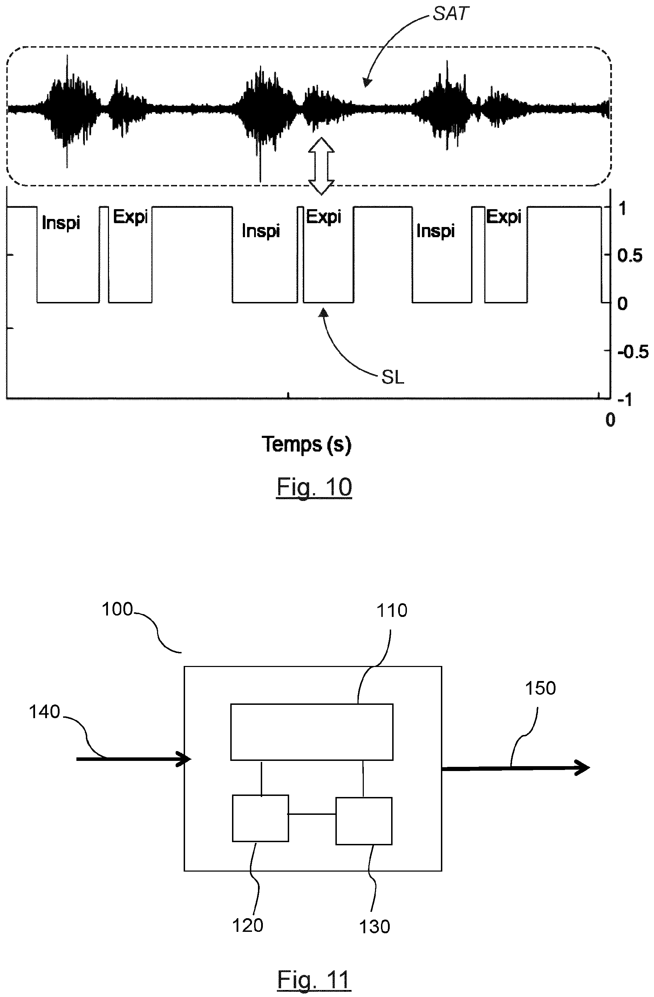

La **figure 1** représente, de manière générique, un organigramme d'un mode de réalisation particulier du procédé selon l'invention. Cet organigramme illustre les étapes principales de mise en œuvre du procédé, notées S1 à S4. Ces étapes sont mises en œuvre par un dispositif (dont le principe est décrit plus loin en relation avec la figure 11) et visent à déterminer les phases d'inspiration et d'expiration d'une activité respiratoire d'un individu.

La **figure 2** présente un individu X, en position allongée, soumis à un examen respiratoire, par exemple une polygraphie ventilatoire. L'individu X présente au niveau de sa trachée un capteur acoustique 1, maintenu par un adhésif médical. Un tel capteur 1 est configuré pour capter des ondes de pression sonore provenant de la trachée de l'individu X pendant son sommeil. Ces ondes de pression sonore sont transformées par le capteur en signaux électriques analogiques, ces derniers étant ensuite convertis en signaux numériques avant d'être transmis à une unité de traitement 2. L'unité de traitement 2 est reliée au capteur 1 via une liaison électrique 3 (ou via une liaison sans-fil à courte-portée par exemple). Afin de permettre un traitement sur une plage de fréquences suffisamment grande, le capteur 1 est configuré de manière à présenter une bande passante s'étendant typiquement entre 10 Hz et 10kHz. Bien entendu, cette plage de fréquences est donnée à titre d'exemple illustratif et d'autres plages peuvent être envisagées sans sortir du cadre de l'invention en fonction de l'application visée ou de l'examen pratiqué. L'unité de traitement 2, quant à elle, est configurée pour recevoir, stocker et traiter les signaux transmis par le capteur 1. Elle possède les instructions de code de programme permettant la mise en œuvre du procédé de la présente invention. Une interface homme-machine reliée à l'unité de traitement permet à l'utilisateur (au personnel médical en l'occurrence) de suivre l'évolution de l'activité respiratoire de l'individu X et d'exécuter le procédé de la présente invention à des fins d'analyse et de diagnostic.

Dans la suite du document, on entend par « signal acoustique », les signaux électriques représentatifs de l'activité respiratoire de l'individu X qui ont été collectés et enregistrés par l'unité de traitement 2 au cours de l'examen.

L'activité respiratoire d'un individu est typiquement composée de cycles respiratoires, chaque cycle comprenant une phase d'inspiration et une phase d'expiration qui produisent des ondes de pression sonore au sein de l'individu. La phase d'inspiration correspond à un remplissage des poumons pendant laquelle le diaphragme et les muscles intercostaux se contractent. La phase d'expiration correspond à un vidage des poumons pendant laquelle les muscles thoraciques sont relâchés.

Le procédé est initialisé par activation de l'enregistrement de l'activité respiratoire de l'individu X ou après une durée prédéterminée d'enregistrement.

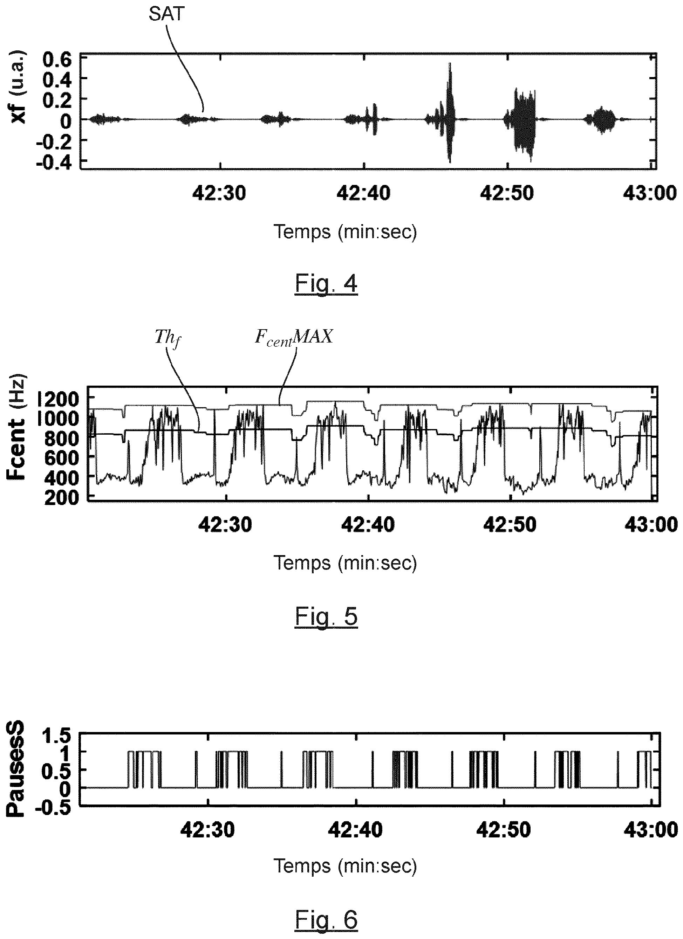

A l'étape **S1** (référencée « OBT_S »), le dispositif acquiert le signal acoustique trachéal de l'individu X. Un exemple de signal acoustique trachéal (référencé « SAT ») est représenté sur la **figure 4.**

A l'étape **S2** (référencée « DET_PA »), le dispositif procède, par analyse de signal, à une détection des intervalles de pause contenus dans le signal SAT.

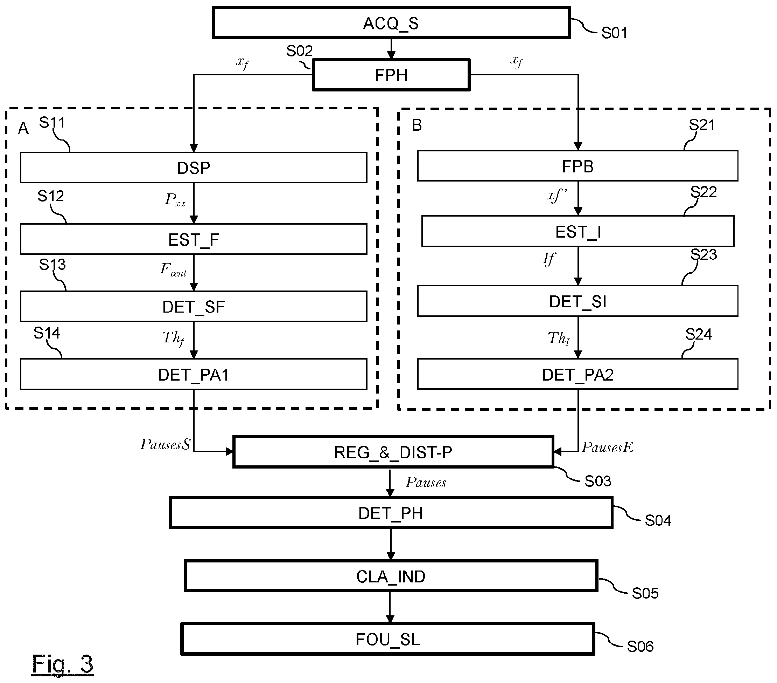

Cette étape de détection repose sur la mise en œuvre, par le dispositif, d'un traitement fréquentiel du signal SAT et/ou d'un traitement énergétique du signal SAT. Le principe de ces deux traitements est décrit en détail ci-après en relation avec les blocs A et B de l'organigramme de la **figure 3**. Les intervalles de pause détectés sont stockés dans une table locale du dispositif.

On entend par « intervalle de pause », un intervalle de temps au cours duquel la portion de signal correspondante est représentative d'une pause respiratoire, autrement dit d'une absence d'activité respiratoire. Cette absence d'activité respiratoire caractéristique d'un cycle d'une activité respiratoire classique se repère aisément dans le signal tant dans le domaine fréquentiel que dans le domaine énergétique.

A l'étape **S3** (référencée « DIS_P »), le dispositif effectue une distinction, parmi les intervalles de pause détectés à l'étape S2, entre un premier type d'intervalle de pause, appelé « pause courte » et un deuxième type d'intervalle de pause, appelé « pause longue », en fonction de la durée estimée pour chacun des intervalles pause. On considère ici qu'un intervalle de type « pause courte » est de durée inférieure à un intervalle de type « pause longue ».

Selon une mise en œuvre particulière, après avoir estimé la durée des intervalles de pause détectés, le dispositif procède à une comparaison de durée des intervalles de pause qui se succèdent deux-à-deux dans le temps pour déterminer laquelle est la pause courte et laquelle est la pause longue.

De manière complémentaire ou alternative, le dispositif compare la durée de chacun des intervalles de pause en fonction d'un seuil prédéterminé. Un tel seuil consiste en une durée choisie pour différencier les pauses longues des pauses courtes parmi les intervalles de pause détectés. On considère par exemple qu'un intervalle de pause correspond à une pause longue si la durée estimée pour cet intervalle est supérieure audit seuil. Sinon, il correspond à une pause courte si la durée estimée pour cet intervalle est inférieure audit seuil.

A l'étape **S4** (référencée « DET_PH »), le dispositif détermine les intervalles de temps du signal SAT correspondant aux phases d'inspiration et d'expiration en tenant compte de la règle d'ordonnancement suivante : un intervalle de temps correspond à une phase d'inspiration s'il est compris entre une pause longue et une pause courte, et à une phase d'expiration s'il est compris entre une pause courte et une pause longue. En effet, dans un cycle respiratoire en situation normale de repos, la phase d'inspiration est suivie d'une pause d'inspiration (ou pause courte), laquelle est suivie d'une phase d'expiration, elle-même suivie d'une pause d'expiration (ou pause longue), la pause courte étant de durée inférieure à la pause longue. Autrement dit, le principe repose sur la comparaison, pour chaque intervalle de temps compris entre deux pauses successives, de la pause qui précède et de la pause qui suit ledit intervalle. Une inspiration est suivie d'une pause plus courte que celle qui la précède, une expiration est suivie d'une pause plus longue que celle qui la précède. C'est en partant de ce constat que la règle d'ordonnancement précitée a été établie pour la présente invention.

Ainsi, à l'issue de l'étape S4 du procédé, le dispositif dispose d'une segmentation du signal SAT en intervalles de temps associés chacun à l'une des trois catégories suivantes : une phase d'inspiration, une phase d'expiration, une pause. Ces informations peuvent être fournies sous la forme d'un signal logique transitoire représentatif de l'activité respiratoire de l'individu X, dont les états logiques correspondent aux phases et aux pauses respiratoires dudit individu. Le dispositif communique à l'interface homme-machine le signal logique représentatif de l'activité respiratoire de l'individu X, à des fins d'affichage et de diagnostic par le personnel médical.

Ainsi, le principe général du procédé repose sur la recherche dans un signal acoustique trachéal de pauses de l'activité respiratoire d'un individu pour en déduire les phases d'inspiration et d'expiration contenus dans ce signal. Cette technique de reconnaissance de phases respiratoires est particulièrement efficace car elle s'attache à traiter prioritairement des portions de signal correspondant à des pauses respiratoires, aisément reconnaissables dans les domaines fréquentiel et énergétique. C'est en observant en effet l'évolution temporelle de signaux acoustiques trachéaux dans les domaines fréquentiel et énergétique que les inventeurs de la présente ont remarqué que les pauses se repèrent plus facilement que les phases d'inspiration et d'expiration en tant que telles. En offrant la possibilité de déterminer les phases inspiratoires et expiratoires uniquement à l'aide de sons trachéaux captés sur un individu, le procédé de l'invention ouvre ainsi des perspectives d'examen simplifié et moins invasif, requérant de plus un équipement médical de diagnostic plus léger.

On présente maintenant, en relation avec la **figure 3,** une implémentation particulière du procédé dans laquelle les étapes S2, S3 et S4 de l'organigramme décrit ci-dessus sont plus amplement détaillées.

Après avoir récupéré le signal acoustique trachéal SAT à l'étape **S01** (référencée « ACQ_S »), le dispositif applique tout d'abord un filtre passe-haut anti-bruit au signal à l'étape **S02** (référencée « FPH »), par exemple un filtre de type Butterworth d'ordre 4 ayant une fréquence de coupure égale à 100 Hz. Ce filtre passe-haut a pour fonction de retirer au signal SAT une plage de fréquences prédéfinie correspondant aux bruits cardiovasculaires émis par l'individu X à travers sa trachée. Le signal obtenu en sortie du filtre passe-haut est un signal filtré dans le domaine temporel, appelé par la suite signal filtré *'xf'*.

L'observation de la représentation temporelle fréquence-énergie a permis de constater que c'est sur ce type de représentation que les pauses de l'activité respiratoire sont les plus faciles à reconnaître. Deux grandeurs physiques distinguent les pauses des phases respiratoires (inspiration/expiration) : le contenu fréquentiel et l'énergie. Il apparaît en effet que l'énergie du signal acoustique trachéal est minimale pendant les pauses puisqu'elle correspond à une absence de respiration et que le spectre du signal correspondant à une pause se rapproche de celui d'un bruit blanc dans la bande de fréquences comprise entre 100 et 2 000 Hz.

Il est proposé deux techniques particulières pour procéder au traitement du signal filtré *'xf'* : un traitement fréquentiel (représenté par le bloc en pointillés A) et un traitement énergétique (représenté par le bloc en pointillés B). La deuxième technique est décrite postérieurement.

> **Traitement fréquentiel du signal**

A l'étape **S11**, le dispositif procède à une segmentation du signal filtré *xf* pour obtenir une pluralité d'échantillons de ce signal. Par exemple, le signal filtré *xf* est divisé en segments de 200 échantillons chacun correspondant à 50 ms. Chaque segment, noté *'l'*, est en outre découpé en trois sous-segments qui se chevauchent à 50%. Puis, le dispositif estime la densité spectrale de puissance (aussi connue sous le nom de « DSP ») de chaque segment *l* de manière à transformer le signal filtré *xf* en un signal fréquentiel, noté *'$F_{cent}$'*, dans le domaine temporel.

La DSP estimée du segment *l*, notée *'$P_{xx}[k,l]$'*, est égale à la moyenne de la DSP des trois sous-segments composant le segment *l*. L'indice *k* représente l'indice de la fréquence *'$F_k$'* tel que $F_k = k.Fs/N_{fft}$, avec la fréquence Fs égale à 4kHz, $N_{fft}$ le nombre de points de la transformée de Fourier (en supposant que $N_{fft}$ est un nombre pair) et *k* un entier compris dans l'intervalle [0 - $N_{fft}/2$]. Pour chaque segment l du signal *xf*, le dispositif estime à l'étape **S12** (« EST_F ») la fréquence centroïde $F_{cent}[l]$ au moyen de la relation suivante :

$$F_{cent}[l] = \frac{\sum_{k=0}^{N_{fft}/2} F_k P_{xx}[k, l]}{\sum_{k=0}^{N_{fft}/2} P_{xx}[k, l]}$$

L'étape **S13** (« DET_SF ») consiste à déterminer un seuil de fréquence variable, noté $Th_f$, qui servira à l'étape S14 pour identifier les intervalles de pause contenus dans le signal acoustique. Pour ce faire, le dispositif définit une fenêtre temporelle glissante de taille prédéfinie correspondant sensiblement à la durée moyenne d'un cycle respiratoire, puis applique cette fenêtre glissante sur le signal fréquentiel $F_{cent}$ pour déterminer le seuil de fréquence dont la valeur est fonction du maximum fréquentiel du signal fréquentiel $F_{cent}$ dans la fenêtre glissante considérée. A titre d'exemple, comme illustré sur la **figure 5,** une fenêtre temporelle de taille prédéfinie correspondant à 80 échantillons (soit une fenêtre de durée sensiblement égale à 4 secondes) est appliquée au signal fréquentiel $F_{cent}$. Pour chaque fenêtre appliquée, le dispositif détermine la valeur maximale de la fréquence du signal fréquentiel $F_{cent}$ de cette fenêtre (notée $F_{centMAX}$ sur la figure), et soustrait à cette valeur maximale une valeur de fréquence prédéfinie ΔF pour en déduire la valeur du seuil de fréquence $Th_f$ pour cette fenêtre. La valeur de fréquence prédéfinie ΔF est comprise typiquement entre 100 Hz et 300 Hz.

Ainsi, plutôt que de définir un seuil de fréquence de valeur prédéterminée, le dispositif selon l'invention propose un calcul de seuil variable se rapprochant au plus près de la forme du signal fréquentiel, ce qui permet de fournir un procédé à fiabilité augmentée.

A noter que la fréquence centroïde est proche de 1000 Hz durant les pauses, ce qui correspond à la fréquence de Nyquist divisée par deux. Ce résultat est cohérent avec l'hypothèse de bruit blanc dans une bande de fréquences comprise entre 100 et 2 000 Hz.

A l'étape **S14** (« DET_PA1 »), le dispositif procède à la mise en forme, à partir du signal fréquentiel $F_{cent}$, d'un signal logique transitoire *PauseS* dont les niveaux logiques sont fonction des valeurs de seuil de fréquence $Th_f$ déterminées à l'étape S13. Un exemple de signal logique *PauseS,* issu d'un traitement fréquentiel, est représenté sur la **figure 6.** Ce signal *PauseS* est représentatif des pauses contenues dans le signal SAT. Ainsi, pour un point donné du signal fréquentiel $F_{cent}$, si la valeur de fréquence associée à ce point est supérieure à la valeur de seuil de fréquence $Th_f$ déterminée pour ce point, alors le niveau logique du signal *PauseS* prend la valeur 1, ce qui signifie qu'un intervalle de pause est détecté. A l'inverse, si la valeur de fréquence associée à ce point est égale ou inférieure à la valeur de seuil de fréquence $Th_f$ déterminée pour ce point, alors le niveau logique du signal *PauseS* prend la valeur 0, ce qui signifie qu'aucun intervalle de pause n'est détecté. Les intervalles de pause détectés sont alors stockés dans la table locale du dispositif. Le traitement fréquentiel prend fin à l'issue de cette étape S14.

> **Traitement énergétique du signal**

Le dispositif applique tout d'abord, à l'étape **S21**, un filtre passe-bas au signal filtré *xf* (référencée « FPB ») afin de retirer les fréquences pour lesquelles le niveau d'intensité sonore trachéal est faible. L'application d'un filtre de type Butterworth d'ordre 6 ayant une fréquence de coupure égale à 1 500 Hz, par exemple, conduit à un signal filtré *xf'* dont la plage de fréquences est comprise entre 100 et 1 500 Hz, correspondant à la bande de fréquences la plus énergétique des sons trachéaux.

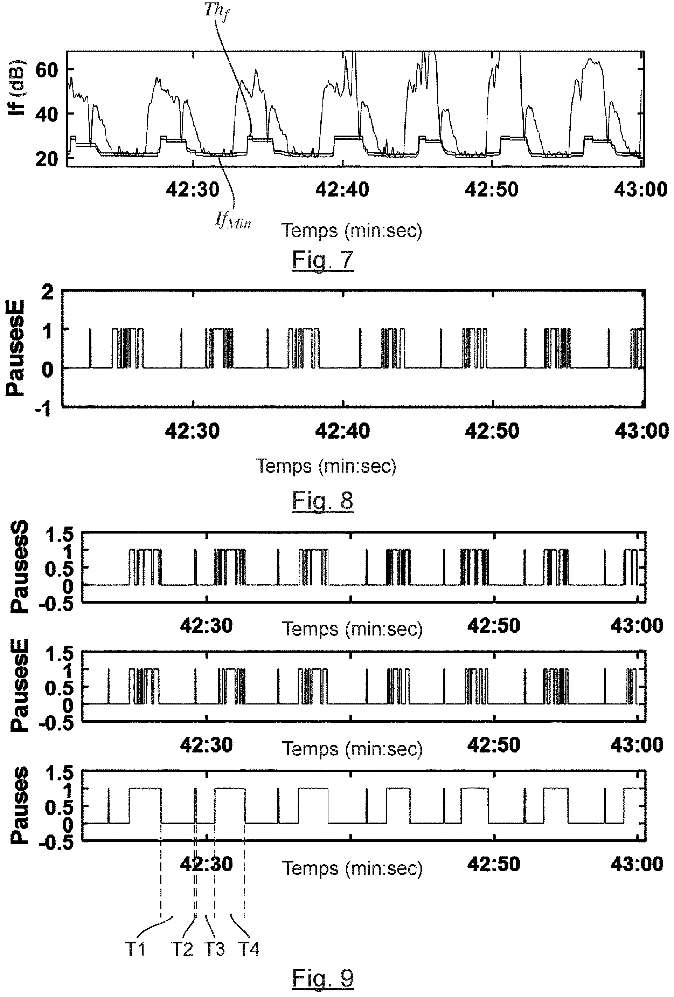

A l'étape **S22** (« EST_I »), le dispositif définit une première fenêtre temporelle glissante de taille prédéfinie, puis applique cette première fenêtre sur le signal filtré *xf'* dans le but d'estimer l'intensité acoustique de ce signal filtré. A titre d'exemple, comme illustré sur la **figure 7,** une enveloppe à moyenne quadratique ou enveloppe RMS *(pour « Root Mean Square »)* ou encore enveloppe à racine carrée de la moyenne des carrés, est calculée à partir du signal filtré $xf'$ en utilisant une fenêtre temporelle glissante de taille égale à 125 échantillons par exemple (soit une fenêtre de durée sensiblement égale à 0,031 s), de façon à obtenir un signal énergétique, noté $'I_f'$, évoluant dans le domaine temporel (le niveau d'intensité acoustique du signal filtré $xf'$ étant proportionnel à la grandeur de l'enveloppe RMS calculée à partir de ce signal).

L'étape **S23** (« DET_SI ») consiste à déterminer le seuil d'intensité variable, noté $Th_I$, qui servira à l'étape S24 pour identifier les intervalles de pause contenus dans le signal acoustique.

Pour ce faire, le dispositif détermine une deuxième fenêtre temporelle glissante, mais cette fois-ci de taille variable W en fonction de la période respiratoire $P_R$. La période respiratoire $P_R$ est elle-même déterminée au moyen d'une fonction d'autocorrélation, notée $r_{I_f}$, appliquée au signal énergétique $I_f$ à l'aide de l'équation suivante :

$$r_{I_f}[l] = \frac{1}{N} \sum_{k=0}^{N-1} I_f[k] I_f[k+l]$$

avec :

k, un entier compris entre 0 et N - 1,
N, le nombre de points de la fenêtre d'autocorrélation, avec N = 240 000 (correspondant à 60 secondes à la fréquence d'échantillonnage Fs = 4 kHz),
l, un entier compris entre (-N + 1) et (N -1).

Pour $l \geq 2000$ (soit 1/2 s), la période respiratoire $P_R$ est déduite à partir de la localisation du maximum de la fonction $r_{I_f}[l]$. Ensuite, on considère que la taille W de la deuxième fenêtre temporelle glissante représente une fraction, inférieure à 0,5, de la période respiratoire $P_R$ (par exemple 0,4 x $P_R$) afin de tenir compte de la plus petite durée qu'une phase d'inspiration peut présenter. On commence la recherche à 1/2 seconde car la fonction d'autocorrélation est maximum lorsque $l = 0$.

Puis, le dispositif applique les deuxièmes fenêtres glissantes déterminées ci-dessus sur le signal énergétique $I_f$ en vue d'identifier la valeur minimale de l'intensité sonore du signal énergétique $I_f$ (notée $I_{Min}$) apparaissant pour chaque deuxième fenêtre glissante déterminée ci-dessus. Pour se faire, le dispositif recherche, pour un instant donné du signal, le minimum d'intensité sonore 0,2 x $P_R$ avant et 0,2 x $P_R$ après ledit instant. Puis, pour chaque fenêtre glissante considérée, est ajoutée à la valeur minimale d'intensité sonore déterminée pour ladite fenêtre considérée, une valeur d'intensité prédéfinie $\Delta I$ de manière à déduire la valeur de seuil d'intensité à prendre en compte pour ladite fenêtre considérée. La valeur d'intensité prédéfinie $\Delta I$ est comprise typiquement entre 1 et 2 dB.

Ainsi, le dispositif de l'invention propose un calcul de seuil variable se rapprochant au plus près de la forme du signal énergétique, ce qui permet de fournir un procédé à fiabilité augmentée.

A l'étape **S24** (« DET_PA2 »), le dispositif procède à la mise en forme, à partir du signal énergétique $I_f$, d'un signal logique transitoire *PauseE* dont les niveaux logiques sont fonction des valeurs de seuil d'intensité $Th_I$ déterminées à l'étape S13. Un exemple de signal logique *PauseE* est représenté sur la **figure 8.** Comme pour le signal logique *PauseS,* ce signal *PauseE* est représentatif des pauses contenues dans le signal SAT. Ainsi, pour un point donné du signal $I_f$, si le niveau d'intensité sonore associé à ce point est inférieur au seuil d'intensité déterminé pour ce point, alors le niveau logique du signal *PauseE* prend la valeur 1, signifiant qu'un intervalle de pause est détecté. A l'inverse, si le niveau d'intensité associé à ce point est supérieur ou égal au seuil d'intensité déterminé pour ce point, alors le niveau logique du signal *PauseE* prend la valeur 0, signifiant qu'aucun intervalle de pause n'est détecté.

Les intervalles de pause ainsi détectés sont alors stockés dans la table locale du dispositif. Le traitement fréquentiel prend fin à l'issue de cette étape S24.

A la fin des étapes de traitement S14 et S24, le dispositif peut décider de retourner les chronogrammes des signaux logiques *PauseE* et *PauseS* courants à l'utilisateur via l'interface homme-machine, afin de mettre en évidence les pauses respiratoires contenues dans le signal SAT via le traitement fréquentiel et le traitement énergétique.

A l'étape **S03** (« REG_&_DIST-P »), le dispositif dispose d'un premier ensemble d'intervalles de pause issues du traitement fréquentiel (chronogramme du signal logique *PauseS*) et d'un deuxième ensemble d'intervalles de pause issues du traitement énergétique (chronogramme du signal logique *PauseE*). Le dispositif va alors traiter les signaux logiques *PauseS et PauseE* par une fonction logique 'OU-Inclusif' de manière à obtenir un signal logique final *'Pause'* dont les niveaux logiques hauts correspondent aux intervalles de pause détectés. Comme illustré sur la **figure 9,** en cas de présence d'un niveau logique de valeur 1 pour au moins l'un des signaux logiques *PauseS et PauseE,* le niveau logique du signal final prend la valeur 1 (signifiant qu'un intervalle de pause est détecté), sinon le niveau logique du signal final prend la valeur 0 (signifiant qu'aucun intervalle de pause est détecté). Le dispositif procède ainsi à un regroupement des intervalles de pause issus des deux chronogrammes *PauseS et PauseE.*

Ainsi, comme décrit précédemment, le mode de réalisation décrit ici repose sur une mise en œuvre simultanée des deux traitements de signal. Un tel mode de réalisation rend le procédé encore plus fiable et robuste. Il est tout à fait envisageable, à titre d'alternative, de ne mettre en œuvre qu'un seul des deux traitements précités pour repérer les intervalles de pause dans le signal acoustique, par exemple pour gagner en rapidité de traitement ou encore parce que l'un des deux traitements est plus adapté à la nature du signal acoustique étudié.

Le dispositif vérifie dans un premier temps si des successions d'intervalles de pause séparées par un intervalle de temps - dit intervalle aberrant - de durée inférieure à un seuil prédéterminé sont présentes dans le signal logique obtenu à l'issue de l'étape S03. La valeur de ce seuil prédéterminé est choisie pour être de très courte durée, c'est-à-dire une durée typiquement inférieure à 1/4 s. Ainsi, un intervalle de temps compris entre deux pauses de durée inférieure au seuil prédéterminé est considéré par le dispositif comme une aberration du signal et doit faire partie d'un unique intervalle de pause. En cas de vérification positive pour deux pauses successives données, l'intervalle de temps regroupant les deux pauses de ladite succession et l'intervalle aberrant est remplacé, dans le signal logique final par un nouvel intervalle de pause (de niveau logique 1). En cas de vérification négative, aucun remplacement n'est opéré.

A l'étape suivante **S04** (« DET_PH »), le dispositif détermine les phases d'inspiration et d'expiration du signal acoustique en comparant, pour chaque intervalle de temps restant, la durée de la pause qui précède relativement à la durée qui suit ledit intervalle. Le dispositif considère qu'un intervalle de temps du signal de niveau logique bas correspond à :

- une phase d'inspiration s'il est compris entre une pause longue et une pause courte ; ou
- une phase d'expiration s'il est compris entre une pause courte et une pause longue.

En effet, on rappelle que dans un cycle respiratoire typique, une inspiration est précédée d'une pause longue et suivie d'une pause courte et une expiration est précédée d'une pause courte et suivie d'une pause longue.

Une simple comparaison de la durée relative des pauses précédant et suivant chaque intervalle de temps est ainsi réalisée par le dispositif. Il n'est donc pas nécessairement utile de mesurer la durée puis d'allouer un état de durée en fonction de cette mesure aux pauses détectées. A titre d'alternative, il est possible de configurer le dispositif pour qu'il calcule la valeur absolue de la durée des pauses détectées, puis de procéder à une comparaison des valeurs calculées pour déduire les phases d'expiration et d'inspiration du signal.

Ces informations sont stockées dans une table locale du dispositif.

Comme illustré sur la figure 9, à l'étape **S04,** le dispositif sait, par comparaison des durées, que la pause T2 du signal *Pauses* est une pause de type courte et que la pause T4 est une pause de type longue. Comme une phase expiration est précédée d'une pause courte et suivie d'une pause longue, il en déduit, à l'étape S04, que l'intervalle d'activité respiratoire T3 correspond à une phase d'expiration.

L'étape S05 (« CLA_IND") consiste à améliorer davantage l'algorithme de détermination selon l'invention en vérifiant si, pour certains intervalles de temps, la phase respiratoire à laquelle ils sont alloués, ne serait pas incohérente avec un cycle respiratoire classique. Cette étape est réalisée en fonction d'un ou plusieurs critères d'incohérences prédéterminés relatif au cycle respiratoire humain. Une succession d'au moins deux phases d'inspiration ou d'expiration constitue un exemple de critère d'incohérence applicable à la présente invention. Les cycles qui ne respectent pas les critères de cohérence sont alors classés comme indéterminés. L'Homme du Métier est à même d'adapter la liste des critères d'incohérence possibles et de les combiner éventuellement en fonction du poids qu'il souhaite donner à cette étape de l'algorithme. En cas de vérification positive pour un intervalle donné, ce dernier est considéré comme un « intervalle indéterminé ». Pour chaque intervalle indéterminé du signal, le dispositif traite la portion de signal correspondant audit intervalle indéterminé au moyen d'une méthode d'apprentissage automatique supervisée, telle que par exemple la méthode connue sous le nom de *« Random forest »* basée sur des arbres de décision aléatoire. Cette méthode est adaptée à chaque patient en réalisant la phase d'apprentissage à l'aide des cycles supposés bien classés de celui-ci. Les caractéristiques sont extraites du signal filtré *xf,* par exemple la durée, l'énergie, la puissance, la forme, le contenu spectrale, ou tout autre caractéristique que l'homme du métier pourra déterminer.

De manière complémentaire ou alternative, un simple traitement des intervalles indéterminés basé sur la cohérence des successions des phases respiratoires et des durées connues des phases d'inspiration et d'expiration, peut être mis en œuvre antérieurement ou simultanément à la méthode d'apprentissage pour permettre de reclasser tout ou partie des intervalles indéterminés de manière simple et rapide.

Grâce à cette étape, on affine ainsi davantage le mécanisme de reconnaissance des phases d'inspiration et d'expiration lorsque certaines phases déterminées répondent audit critère d'incohérence.

Enfin, à l'étape **S06** (« FOU_SL »), le dispositif fournit, via l'interface homme-machine, les informations relatives aux phases respiratoires préalablement déterminées sous forme d'un signal logique transitoire apte à pouvoir être interprété par un personnel médical qualifié. Comme illustré à titre d'exemple sur le chronogramme de la **figure 10,** un tel signal logique SL présente un niveau logique bas (de valeur égale à 0) lorsqu'il s'agit d'une phase d'inspiration (notée « Inspi » sur la figure) ou d'une phase expiration (notée « Expi »), et un niveau logique haut (de valeur égale à 1) lorsqu'il s'agit d'une pause. Les paramètres associés aux phases d'inspiration et d'expiration de l'activité respiratoire de l'individu X peuvent être déduit à partir de ce signal logique SL à des fins de diagnostic.

La **figure 11** présente la structure simplifiée d'un dispositif 100 mettant en œuvre le procédé de détermination selon l'invention (par exemple le mode de réalisation particulier décrit ci-dessus en relation avec les figures 1 à 10). Ce dispositif comprend une mémoire vive 130 (par exemple une mémoire RAM), une unité de traitement 110, équipée par exemple d'un processeur, et pilotée par un programme d'ordinateur stocké dans une mémoire morte 120 (par exemple une mémoire ROM ou un disque dur). A l'initialisation, les instructions de code du programme d'ordinateur sont par exemple chargées dans la mémoire vive 130 avant d'être exécutées par le processeur de l'unité de traitement 110. L'unité de traitement 110 reçoit en entrée le signal acoustique trachéal 140 (par exemple le signal SAT). Le processeur de l'unité de traitement 110 traite le signal 140 selon les instructions du programme d'ordinateur et génère en sortie 150 un signal logique transitoire final mettant en évidence les différentes phases respiratoires de l'individu. Ce traitement est réalisé à l'aide de moyens de détection d'intervalles de pause, de moyens de distinction de pauses longues ou de pauses courtes et de moyens de détermination de phases respiratoires en fonction des pauses détectées.

Cette figure 11 illustre seulement une manière particulière, parmi plusieurs possibles, de réaliser les différents algorithmes détaillés ci-dessus, en relation avec les figures 1 et 3. En effet, la technique de l'invention se réalise indifféremment :

- sur une machine de calcul reprogrammable (un ordinateur PC, un processeur DSP ou un microcontrôleur) exécutant un programme comprenant une séquence d'instructions, ou
- sur une machine de calcul dédiée (par exemple un ensemble de portes logiques comme un FPGA ou un ASIC, ou tout autre module matériel).

Dans le cas où l'invention est implantée sur une machine de calcul reprogrammable, le programme correspondant (c'est-à-dire la séquence d'instructions) pourra être stocké dans un médium de stockage amovible (tel que par exemple une disquette, un CD-ROM ou un DVD-ROM) ou non, ce médium de stockage étant lisible partiellement ou totalement par un ordinateur ou un processeur.

Le mode de réalisation particulier présenté ci-dessus repose sur le traitement d'un signal acoustique trachéal. Bien entendu, il est tout à fait possible d'appliquer le procédé de l'invention sur un signal acoustique provenant d'un autre organe de l'individu, tel qu'un signal acoustique pulmonaire par exemple (pour ne citer qu'un exemple d'application). De manière plus générale, la technique de l'invention s'applique à tout type de signaux acoustiques représentatifs de l'activité respiratoire d'un individu. L'invention et ses modes de réalisation préférés sont définis dans les revendications annexées.

## Revendications

1. Procédé de détermination de phases respiratoires dans un signal acoustique représentatif d'une activité respiratoire d'un individu, l'activité respiratoire étant définie par des cycles respiratoires chacun comprenant une phase d'inspiration et une phase d'expiration, le procédé comprenant une étape de détection (S2) d'intervalles de pause dans le signal acoustique et une étape de détermination (S4) d'intervalles de temps correspondant à des phases d'inspiration et d'intervalles de temps correspondant à des phases d'expiration par comparaison, pour chaque intervalle de temps, de la durée de l'intervalle de pause qui précède ledit intervalle de temps avec la durée de l'intervalle de pause qui suit ledit intervalle de temps,
le procédé étant **caractérisé en ce qu'**il comprend les étapes suivantes, si à l'issue de l'étape de détermination (S4) au moins un intervalle de temps, dit intervalle indéterminé, répondant à un critère d'incohérence prédéterminé, est identifié :

   - traitement, pour chaque intervalle indéterminé, de la portion de signal correspondant audit intervalle indéterminé, au moyen d'une méthode d'apprentissage automatique ;
   - association dudit au moins un intervalle indéterminé à une phase d'inspiration ou d'expiration en fonction des résultats de l'étape de traitement.

2. Procédé selon la revendication 1, dans lequel est effectuée, si à l'issue de l'étape de détection (S2) au moins une succession de deux intervalles de pause séparés dans le signal par un intervalle de temps, dit intervalle aberrant, de durée inférieure à un seuil de durée prédéterminé est identifiée :

   - une étape de remplacement, pour chaque succession détectée, de l'ensemble constitué par les deux intervalles de pause et ledit intervalle aberrant par un nouvel intervalle de pause.

3. Procédé selon l'une quelconque des revendications 1 et 2, dans lequel l'étape de détection (S2) d'intervalles de pause repose sur un traitement appartenant au groupe comprenant :

   - un traitement fréquentiel du signal acoustique ;
   - un traitement énergétique du signal acoustique.

4. Procédé selon la revendication 3, dans lequel le traitement fréquentiel comprend les étapes suivantes :

   - application d'un filtre passe-haut anti-bruit audit signal pour retirer une première plage prédéfinie de fréquences audit signal et obtenir un signal filtré en fonction du temps ;

- segmentation dudit signal filtré pour obtenir une pluralité d'échantillons dudit signal filtré et, pour chaque échantillon, estimation de densité spectrale moyenne de puissance dudit échantillon de manière à obtenir un signal fréquentiel représentant la fréquence centroïde en fonction du temps ;

- application d'une fenêtre temporelle glissante de durée prédéterminée sur ledit signal fréquentiel et, pour chaque fenêtre :

* détection d'un maximum fréquentiel du signal fréquentiel dans ladite fenêtre,
* détermination d'un seuil de fréquence en fonction du maximum fréquentiel détecté dans ladite fenêtre ;

- mise en forme, à partir dudit signal fréquentiel, d'un premier signal logique transitoire de niveaux logiques définis en fonction des seuils de fréquence déterminés pour ledit signal fréquentiel, les intervalles de pauses étant détectés en fonction du premier signal logique transitoire.

5. Procédé selon l'une quelconque des revendications 3 et 4, dans lequel le traitement énergétique comprend les étapes suivantes :

- application d'un filtre passe-haut anti-bruit et d'un filtre passe-bas pour retirer audit signal respectivement une première et une deuxième plages prédéfinies de fréquences et obtenir un signal filtré en fonction du temps ;

- application d'une première fenêtre temporelle glissante de durée prédéterminée sur ledit signal filtré et, pour chaque fenêtre temporelle, estimation de l'intensité acoustique dudit signal filtré par application d'une enveloppe à moyenne quadratique dans ladite fenêtre, de manière à transformer ledit signal filtré en un signal énergétique en fonction du temps ;

- application d'une deuxième fenêtre temporelle glissante de durée déterminée par autocorrélation sur ledit signal énergétique et, pour chaque fenêtre :

* détection d'un minimum d'intensité du signal énergétique dans ladite fenêtre,
* détermination d'un seuil d'intensité déterminé en fonction du minimum d'intensité détecté dans ladite fenêtre ;

- mise en forme, à partir dudit signal énergétique, d'un deuxième signal logique transitoire de niveaux logiques définis en fonction des seuils d'intensité déterminés pour ledit signal énergétique, les intervalles de pauses étant détectés en fonction du deuxième signal logique transitoire.

6. Procédé selon la revendication 5, comprenant une étape de mise en forme, par traitement desdits premier et deuxième signaux logiques transitoires par une fonction logique OU-Inclusif, d'un signal logique final de niveaux logiques hauts correspondant auxdits intervalles de pause détectés.

7. Procédé selon la revendication 6, comprenant une étape consistant à, si à l'issue de l'étape de mise en forme, au moins une succession de deux intervalles de pause séparés par un intervalle aberrant détectée dans ledit signal logique final de durée inférieure au seuil de durée prédéterminée est identifiée :

- remplacer, pour chaque succession détectée dans ledit signal logique final, de l'ensemble constitué par les deux intervalles de pause et ledit intervalle aberrant par un nouvel intervalle de pause.

8. Produit programme d'ordinateur, comprenant des instructions de code de programme pour la mise en œuvre du procédé selon au moins une des revendications 1 à 7, lorsque ledit programme est exécuté sur un ordinateur.

9. Médium de stockage lisible par ordinateur et non transitoire, stockant un produit programme d'ordinateur selon la revendication 8.

10. Dispositif de détermination de phases respiratoires dans un signal acoustique représentatif d'une activité respiratoire d'un individu, l'activité respiratoire étant définie par des cycles respiratoires chacun comprenant une phase d'inspiration et une phase d'expiration, le dispositif comprenant :

- des moyens de détection d'intervalles de pause dans le signal acoustique ;
- des moyens de détermination d'intervalles d'activité correspondant à des phases d'inspiration et d'intervalles d'activité correspondant à des phases d'expiration tenant compte par comparaison de la durée des intervalles de pause ;

le dispositif étant **caractérisé en ce qu'**il comprend :

- des moyens de traitement de la portion de signal correspondant d'au moins un intervalle indéterminé au moyen d'une méthode d'apprentissage automatique, un intervalle indéterminé étant un intervalle de temps déterminé répondant à un critère d'incohérence prédéterminé ;
- des moyens d'association dudit au moins un intervalle indéterminé à une phase d'inspiration ou d'expiration en fonction des résultats issus des moyens de traitement.

**Patentansprüche**

1. Verfahren zum Bestimmen von Atmungsphasen in einem akustischen Signal, repräsentativ für eine Atmungstätigkeit einer Person, wobei die Atmungstätigkeit durch Atmungszyklen definiert ist, von denen jeder eine Einatmungsphase und eine Ausatmungsphase umfasst, wobei das Verfahren umfassend einen Schritt zum Erkennen (S2) von Pausenintervallen im akustischen Signal und einen Schritt zum Bestimmen (S4) von Zeitintervallen entsprechend den Einatmungsphasen und von Zeitintervallen entsprechend den Ausatmungsphasen, durch Vergleichen, für jedes Zeitintervall, der Dauer des Pausenintervalls, welches dem Zeitintervall vorangeht, mit der Dauer des Pausenintervalls, welches dem Zeitintervall nachfolgt, wobei das Verfahren **dadurch gekennzeichnet ist, dass** es, wenn am Ende des Schrittes zum Bestimmen (S4) mindestens ein Zeitintervall, unbestimmtes Intervall genannt, welches einem vorbestimmten Inkohärenz-Kriterium entspricht, identifiziert wird, die folgenden Schritte umfasst;

   - Bearbeiten, für jedes unbestimmte Intervall, des Signalabschnitts entsprechend dem unbestimmten Intervall anhand einer automatischen Lernmethode;
   - Zuordnung des mindestens einen unbestimmten Intervalls zu einer Ein- oder Ausatmungsphase je nach den Ergebnissen des Schrittes zum Bearbeiten.

2. Verfahren nach Anspruch 1, bei dem, wenn am Ende des Schrittes zum Erfassen (S2) mindestens einer Aufeinanderfolge von zwei getrennten Pausenintervallen in dem Signal durch ein Zeitintervall, abweichendes Intervall genannt, mit einer Dauer geringer als eine vorbestimmte Dauergrenze identifiziert wird, Folgendes ausgeführt wird:

   - ein Schritt zum Ersetzen, für jede erkannte Aufeinanderfolge, der Einheit bestehend aus den beiden Pausenintervallen und dem abweichenden Intervall, durch ein neues Pausenintervall.

3. Verfahren nach einem der Ansprüche 1 und 2, wobei der Schritt zum Erfassen (S2) von Pausenintervallen auf einer Bearbeitung beruht, welche der Gruppe angehört, die Folgendes umfasst:

   - eine Frequenzbearbeitung des akustischen Signals;
   - eine Energiebearbeitung des akustischen Signals.

4. Verfahren nach Anspruch 3, wobei die Frequenzbearbeitung die folgenden Schritte umfasst:

   - Anwenden eines Geräuschschutz-Hochpassfilters zum Entfernen eines ersten vorbestimmten Frequenzbereichs aus dem Signal und Erhalten eines gefilterten Signals in Abhängigkeit von der Zeit;
   - Segmentieren des gefilterten Signals zum Erhalten einer Vielzahl von Mustern des gefilterten Signals, und, für jedes Muster Schätzen der mittleren Leistungsspektraldichte des Musters, um ein Frequenzsignal zu erhalten, welches die Schwerpunktfrequenz in Abhängigkeit von der Zeit darstellt;
   - Anwenden eines gleitenden Zeitfensters mit einer vorbestimmten Dauer auf das Frequenzsignal, und für jedes Fenster:

      * Erkennen eines Frequenzhöchstwertes des Frequenzsignals in dem Fenster,
      * Bestimmen einer Frequenzgrenze in Abhängigkeit von dem in dem Fenster erkannten Frequenzhöchstwert;

   - in Form bringen aus dem Frequenzsignal eines ersten logischen Übergangssignals mit Logikpegeln, welche in Abhängigkeit von den bestimmten Frequenzgrenzen für das Frequenzsignal definiert werden, wobei die Pausenintervalle in Abhängigkeit von dem ersten logischen Übergangssignal erkannt werden.

5. Verfahren nach einem der Ansprüche 3 und 4, wobei die Energiebearbeitung die folgenden Schritte umfasst:

   - Anwenden eines Geräuschschutz-Hochpassfilters und eines Tiefpassfilters zum Entfernen aus dem Signal jeweils eines ersten und zweiten vorbestimmten Frequenzbereichs und Erhalten eines gefilterten Signals in Abhängigkeit von der Zeit;
   - Anwenden eines ersten gleitenden Zeitfensters mit vorbestimmter Dauer auf das gefilterte Signal, und für jedes Zeitfenster Schätzen der Schallintensität des gefilterten Signals durch Anwenden einer Hülle mit quadratischem Mittel in dem Fenster, um das gefilterte Signal in ein Energiesignal in Abhängigkeit von der Zeit umzuwandeln;

- Anwenden eines zweiten gleitenden Zeitfensters mit bestimmter Dauer durch Autokorrelation auf das Energiesignal, und für jedes Fenster:

* Erkennen eines Intensitätsmindestwertes des Energiesignals in dem Fenster,
* Bestimmen einer Intensitätsgrenze in Abhängigkeit von dem in dem Fenster erkannten Intensitätsmindestwert;

- in Form bringen aus dem Energiesignal eines zweiten logischen Übergangssignals von Logikpegeln, welche in Abhängigkeit von den bestimmten Intensitätsgrenzen für das Energiesignal definiert werden, wobei die Pausenintervalle in Abhängigkeit von dem zweiten logischen Übergangssignal erkannt werden.

6. Verfahren nach Anspruch 5, umfassend einen Schritt zum in Form bringen der ersten und zweiten logischen Übergangssignale durch eine logische ODER-inklusive Funktion, eines logischen Endsignals, dessen oberen Logikpegel den erkannten Pausenintervallen entsprechen.

7. Verfahren nach Anspruch 6, umfassend einen Schritt, der, wenn am Ende des Schrittes zum in Form bringen mindestens eine Aufeinanderfolge von zwei durch ein abweichendes Intervall getrennten Pausenintervallen, welches in dem logischen Endsignal mit einer Dauer geringer als die vorbestimmte Dauergrenze erkannt wird, identifiziert wird, darin besteht:

- für jede erkannte Aufeinanderfolge in dem logischen Endsignal, der Einheit bestehend aus den beiden Pausenintervallen und dem abweichenden Intervall, durch ein neues Pausenintervall zu ersetzen.

8. Computerprogrammprodukt, umfassend Programmcodeanweisungen zur Umsetzung des Verfahrens nach mindestens einem der Ansprüche 1 bis 7, wenn das Programm auf einem Computer ausgeführt wird.

9. Speichermedium, computerlesbar und nicht flüchtig, welches ein Computerprogrammprodukt nach Anspruch 8 speichert.

10. Vorrichtung zum Bestimmen von Atmungsphasen in einem akustischen Signal angeboten, welches repräsentativ für eine Atmungstätigkeit einer Person ist, wobei die Atmungstätigkeit durch Atmungszyklen definiert wird, welche jeweils eine Einatmungsphase und eine Ausatmungsphase umfassen, wobei die Vorrichtung umfasst:

- Mittel zum Erkennen von Pausenintervallen in dem akustischen Signal;
- Mittel zum Bestimmen von Tätigkeitsintervallen entsprechend den Einatmungsphasen und Tätigkeitsintervallen entsprechend den Ausatmungsphasen unter Berücksichtigung durch Vergleichen der Dauer der Pausenintervalle;

wobei die Vorrichtung **dadurch gekennzeichnet ist, dass** sie umfasst:

- Mittel zum Bearbeiten, für jedes unbestimmte Intervall, des Signalabschnitts entsprechend eines unbestimmten Intervalls anhand einer automatischen Lernmethode, wobei ein unbestimmtes Intervall ein Zeitintervall ist, welches einem vorbestimmten Inkohärenz-Kriterium entspricht;
- Mittel zur Zuordnung des mindestens einen unbestimmten Intervalls zu einer Ein- oder Ausatmungsphase je nach den Ergebnissen des Bearbeitens.

**Claims**

1. A method for determining respiratory phases in an acoustic signal representative of a respiratory activity of an individual, the respiratory activity being defined by respiratory cycles each comprising an inhalation phase and an exhalation phase, the method comprising a step of detecting (S2) pause intervals in the acoustic signal and a step of determining (S4) time intervals corresponding to inhalation phases and time intervals corresponding to exhalation phases by comparison, for each time interval, of the duration of the pause interval which precedes said time interval with the duration of the pause interval which follows said time interval, the method being **characterised in that** it comprises the following steps: if at the end of the determination step (S4) at least one time interval, called indeterminate interval, meeting a predetermined inconsistency criterion, is identified:

- processing, for each indeterminate interval, the signal portion corresponding to said indeterminate interval, by means of a machine learning method;
- associating said at least one indeterminate interval with an inhalation or exhalation phase depending on the results of the processing step.

2. The method according to claim 1, wherein is performed, if at the end of the detection step (S2) at least one succession of two pause intervals which are separated in the signal by a time interval, called aberrant interval, of duration less than a predetermined duration threshold is identified:

> - a step of replacing, for each detected succession, the set consisting of the two pause intervals and said aberrant interval by a new pause interval.

3. The method according to any one of claims 1 and 2, wherein the step (S2) of detecting pause intervals is based on a processing belonging to the group comprising:

> - a frequency processing of the acoustic signal;
> - an energy processing of the acoustic signal.

4. The method according to claim 3, wherein the frequency processing comprises the following steps:

> - applying a high-pass anti-noise filter to said signal to remove a first predefined frequency range from said signal and obtain a filtered signal as a function of time;
> - segmenting said filtered signal to obtain a plurality of samples of said filtered signal and, for each sample, estimating an average power spectral density of said sample so as to obtain a frequency signal representing the centroid frequency as a function of time;
> - applying a sliding time window of predetermined duration to said frequency signal and, for each window:
>
> > * detecting a frequency maximum of the frequency signal in said window,
> > * determining a frequency threshold depending on the frequency maximum detected in said window;
>
> - shaping, from said frequency signal, a first transient logic signal of logic levels defined depending on the frequency thresholds determined for said frequency signal, the pause intervals being detected depending on the first transient logic signal.

5. The method according to any one of claims 3 and 4, wherein the energy processing comprises the following steps:

> - applying a high-pass anti-noise filter and a low-pass filter to remove respectively first and second predefined frequency ranges from said signal and obtain a filtered signal as a function of time;
> - applying a first sliding time window of predetermined duration to said filtered signal and, for each time window, estimating the acoustic intensity of said filtered signal by applying a root mean square envelope in said window, so as to transform said filtered signal into an energy signal as a function of time;
> - applying a second sliding time window of duration determined by autocorrelation to said energy signal and, for each window:
>
> > * detecting a minimum intensity of the energy signal in said window,
> > * determining an intensity threshold determined as a function of the minimum intensity detected in said window;
>
> - shaping, from said energy signal, a second transient logic signal of logic levels defined depending on the intensity thresholds determined for said energy signal, the pause intervals being detected depending on the second transient logic signal.

6. The method according to claim 5, comprising a step of shaping, by processing said first and second transient logic signals by an OR-Inclusive logic function, a final logic signal of high logic levels corresponding to said detected pause intervals.

7. The method according to claim 6, comprising a step consisting in, if at the end of the shaping step, at least one succession of two pause intervals which are separated by an aberrant interval detected in said final logic signal of duration less than the predetermined duration threshold is identified:

> - replacing, for each succession detected in said final logic signal, the set consisting of the two pause intervals and said aberrant interval by a new pause interval.

8. A computer program product, comprising program code instructions for implementing the method according to at least one of claims 1 to 7, when said program is executed on a computer.

9. A computer-readable and non-transitory storage medium storing a computer program product according to claim 8.

10. A device for determining respiratory phases in an acoustic signal representative of a respiratory activity of an individual, the respiratory activity being defined by respiratory cycles each comprising an inhalation phase and an exhalation phase, the device comprising:

> - means for detecting pause intervals in the acoustic signal;
> - means for determining activity intervals corresponding to inhalation phases and activity intervals corresponding to exhalation phases taking account by comparison of the duration of the pause intervals,

the device being **characterised in that** it comprises:

- means for processing the signal portion corresponding to at least one indeterminate interval, by means of a machine learning method, an indeterminate interval being a time interval determined meeting a predetermined inconsistency criterion;
- means for associating said at least one indeterminate interval with an inhalation or exhalation phase depending on the results of the processing step.

S1

OBT_S

S2

DET_PA

S3

DIS_P

S4

DET_PH

Fig. 1

X

2

3

1

Fig. 2

EP 4 319 642 B1

Fig. 3

Fig. 4

Fig. 5

Fig. 6

Fig. 7

Fig. 8

Fig. 9

Fig. 10

Fig. 11

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 2593007 A